# EUROPEAN PATENT SPECIFICATION

(11) **EP 2 901 151 B1**
(45) Date of publication and mention of the grant of the patent: **16.05.2018**
(21) Application number: 13840799.4
(22) Date of filing: 27.09.2013
(51) Int. Cl.: G01N 33/48, A61B 10/00, G01N 1/10, G01N 33/50, G01N 21/27

(54) **DIAGNOSTIC DEVICES AND METHODS**
DIAGNOSTISCHE INSTRUMENTE UND VERFAHREN
DISPOSITIFS ET PROCÉDÉS DE DIAGNOSTIC

(30) Priority: 27.09.2012 AU 2012904238
(43) Date of publication of application: 05.08.2015
(73) Proprietor: Ellume Pty Ltd., East Brisbane, Queensland 4169 (AU)
(72) Inventor: PARSONS, Sean Andrew, East Brisbane, Queensland 4169 (AU)
(74) Representative: Graham, Emma Louise
(86) International application number: PCT/AU2013/001115
(87) International publication number: WO 2014/047692

(56) References cited:
- WO-A1-96/34287
- WO-A1-2013/036913
- US-A1- 2005 130 120
- US-A1- 2010 135 857
- US-A1- 2011 151 584
- US-B1- 7 044 919
- J. A. SNYDER: "Diagnostic Considerations in the Measurement of Human Chorionic Gonadotropin in Aging Women", CLINICAL CHEMISTRY., vol. 51, no. 10, 11 August 2005 (2005-08-11), pages 1830-1835, XP055265601, WASHINGTON, DC. ISSN: 0009-9147, DOI: 10.1373/clinchem.2005.053595
- ROWE, CA ET AL.: 'An array immunosensor for simultaneous detection of clinical analytes' ANAL. CHEM. vol. 71, 1999, pages 433 - 439, XP000825701

## Description

### Cross-Reference to Related Applications

The present application claims priority from Australian Provisional Patent Application No 2012904238 filed on 27 September 2012.

### Technical Field

The present disclosure relates to devices and methods for identifying conditions in a human or animal body such as pregnancy.

### Background

There exist many types of diagnostic devices for identifying target medical conditions in a human or animal. Increasingly, these devices are being designed for home use. The devices analyse a biological sample from the human or animal, such as a urine sample, blood sample or otherwise, and identify an analyte in the sample that provides a marker for a target condition.

One of the most widely used and recognised diagnostic devices is the home pregnancy test, which commonly employs lateral flow technology and uses human chorionic gonadotropin (hCG) as a marker for pregnancy.

Diagnostic devices that allow highly accurate testing are clearly desirable. Many diagnostic devices provide for binary identification of the target condition, where it is determined only if the target condition is present (a positive result) or not present (a negative result). In these devices, accuracy is a function of the sensitivity of the device, which is its ability to detect true positive results, and the specificity of the device, which is its ability to detect true negative results. Increasing accuracy is particularly important for diagnostic devices used at home, where there can be no trained health professional to interpret identification results and the value of the results to the care of a patient.

The sensitivity of diagnostic devices increases when the devices are configured to detect smaller amounts of the marker analyte, yielding more true positive results. The increase is limited, however, by the increased susceptibility of the device to detecting background amounts of the marker analyte, which may be present naturally in the sample, for example, resulting in a greater number of false positive results. Generally, background production of the marker analyte therefore constitutes "physiological noise" and creates a limit to the usefulness in improvements to the sensitivity of certain diagnostic devices.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

WO 96/34287 describes assays and devices for distinguishing between normal and abnormal pregnancy

### Summary

According to an aspect of the present disclosure there is provided a test device for identifying at least pregnancy in a human or animal body, the test device as set out in the appended claims.

In the preceding and subsequent aspects, identifying the first and/or second analyte in the biological sample may comprise identifying that the first and/or second analyte is present or absent in the sample or identifying a level at which the first and/or second analyte is present in the sample. Similarly, identifying pregnancy may comprise identifying that the first target condition is present or absent in the body or identifying a level at which pregnancy is present in the body. Identification of the level of an analyte in the sample may include identification of an amount of the analyte in all or part of the sample provided. The amount may be determined in a number of ways, e.g. through extrapolation or direct measurement or otherwise, and/or expressed in a number of ways, e.g. as a density, a light intensity, a measure of power intensity or in IU/L, or otherwise.

The second analyte may provide a marker of a second condition of the body, which may be considered a non-target condition or a further target condition, and/or the second analyte may provide an indication of the quality or size of the sample or a component part of the sample.

A condition such as pregnancy may be considered a "target condition" on the basis that the test device and method is adapted to provide information to the user in respect to identification of that condition, e.g. via a display or otherwise. A "target condition" may therefore be a condition about which the user of the test device is intending to discover information. On the other hand a "non-target condition" may be a condition about which the user of the test device has no direct interest. The device may be configured not to display information to the user about the non-target condition, for example.

The term "analyte" is used herein to define any compound or composition to be measured in a sample.

The test device disclosed herein may comprise any one or more capture agents capable of binding specifically to any one of more of the analytes in the sample. Any suitable capture agents may be used. For example, the capture agents may be any one of more agents that have the capacity to bind a relevant species to form a binding pair. Some examples of such binding pairs include, but are not limited to, an antibody (which term encompasses antigen-binding variants or fragments of antibodies, such as Fv, scFv, Fab, Fab1, F(ab')2, domain antibodies (dAbs), "minibodies" and the like, in addition to monoclonal and polyclonal antibodies) and an antigen (wherein the antigen may be, for example, a peptide sequence or a protein sequence); complementary nucleotide or peptide sequences; polymeric acids and bases; dyes and protein binders; peptides and protein binders; enzymes and cofactors, and ligand and receptor molecules, wherein the term receptor refers to any compound or composition capable of recognising a particular molecule configuration, such as an epitopic or determinant site. The capture agent can be an antibody or fragment thereof, which is capable of binding specifically to the analyte of interest.

The test device may be a device that operates as a single unit. The apparatus may be provided in the form of a hand-held device. The test device may be a single-use, disposable, device. Alternatively, the test device may be partly or entirely re-usable. While in some embodiments the test device may be implemented in a laboratory, the test device may designed as a 'point-of-care' device, for home use or use in a clinic, etc. The test device may provide a rapid-test device, with identification of target conditions being provided to the user relatively quickly, e.g., in under 10 minutes, 5 minutes or under 1 minute.

The one or more test portions may be configured for identifying one or more further analytes in the biological sample, e.g. a third analyte different from the first and second analytes. The further analytes may provide markers of further target or non-target conditions of the body and/or provide other indications such as an indication of the quality or size of the sample or a component part of the sample.

The present disclosure recognises that, while identifying the first analyte in the biological sample may provide a prima facie indication of pregnancy in the body, the degree to which the first analyte is present in the sample may have been affected by something other than the first target condition, such as a different condition and/or a quality or size of the sample or part thereof or otherwise. The degree to which it is affected can be correlated at least in part to the degree to which the second analyte is present in the sample. Accordingly, to reduce the possibility that an erroneous determination might be made about at least pregnancy, the apparatus and method can take into account identification of at least the second analyte when identifying the first target condition in consideration of the first analyte. The test device and method may therefore provide for a co-interpretation of the identification of the first and second analytes in order to identify at least the first target condition.

In another embodiment, pregnancy may be identified as being present in the body based on a determination that the first analyte is present at a level above a threshold level in the sample, wherein the threshold level is changed depending on the level of the second analyte present in the sample.

While in the above-described embodiments it is described that the presence, in particular, of pregnancy is identified based on certain criteria, in some embodiments the same criteria may be used to determine the absence of pregnancy in the body. Furthermore, the test device and method may also be configured to determine if identification of pregnancy is not possible. For example, it may be determined that the level at which the second analyte is present in the sample renders any identification of pregnancy based on the first analyte unfeasible. Such a determination may result in a need to carry out identification of pregnancy using different test device or a different method, or to repeat the identification using the same test device or method immediately or at a later stage.

The test device and method may be used to identify a variety of different target conditions using a variety of different types of biological samples and a variety of different first and second analytes. Biological samples may include, for example, blood, serum, plasma, saliva, sputum, urine, ocular fluid, tears, semen, vaginal discharge, nasal secretions and droplets, ear secretions, perspiration, mucus, stool, and/or amniotic, spinal, wound, or abscess fluid. Analytes under test may include any analytes normally present in the biological sample and/or present in the biological sample abnormally, e.g. only as a result of the person providing the sample having one or more specific target or non-target conditions.

hCG is a hormone produced during pregnancy and therefore measurement of the levels of hCG in a biological sample of blood or urine is a well known procedure for testing pregnancy in women. While the levels of hCG rapidly increase after conception, to detect pregnancy very soon after conception, which is highly desirable, test apparatus must be relatively sensitive to low levels of hCG.

Low levels of hCG are present, however, in urine and blood in the general population of women. This background level of hCG varies according to a woman's menstrual cycle and is elevated during the period of ovulation. It is also elevated around the peri-menopause and, to a lesser extent, in post-menopausal women. This creates a problem for pregnancy testing in that the level of hCG indicative of pregnancy in women varies depending on the background level of hCG (the 'physiological noise') that results from their stage in the menstrual cycle and/or their stage in life. While apparatus that is highly sensitive to hCG may be capable of indicating pregnancy very soon after conception, it is more susceptible to yielding false positive indications of pregnancy due to its sensitivity to background noise that can occur as a result of non-pregnant production of hCG known as pituitary hCG. This is clearly undesirable.

The present disclosure recognises, however, that higher levels of LH are present in blood or urine at times when hCG is at higher background levels. For example, an LH surge occurs approximately 24-48 hours before ovulation and LH remains elevated during ovulation. It has been found, for example, that in urine samples provided by women where the level of hCG is >1 IU/L, a level that can provide an early indication of pregnancy, a majority of the women providing the samples were in fact subject to an LH peak. Accordingly, at least until now, identification of pregnancy based on levels of hCG > 1 IU/L has a very high potential to provide false positive indications. The relationship between LH and hCG may be due to cross-reactivity between the hCG assay and the LH assay, which are very similar proteins. However, technical information associated with instruments detecting these proteins has indicated that there may be no cross-reactivity. Indeed, there exists data which suggests that the hCG is made by the anterior pituitary, which is also where LH is synthesised.

Regardless of the reasons for the relationship or correlation between detected levels of hCG and LH in blood or urine, by detecting a relatively high level of LH in a biological sample, it can be determined that the person providing the sample is relatively more likely to provide a false positive indication of pregnancy due to higher background levels of hCG in the sample. Equally, by detecting a relatively low level of LH in a biological sample, it can be determined that the person providing the sample is relatively less likely to provide a false positive indication of pregnancy. Generally, this can allow 'filtering' of the physiological noise, facilitating sensitive and specific identification of pregnancy based on detection of hCG at lower levels than would otherwise be possible. The same filtering approach can be applied to other target conditions and/or using other analytes.

In one embodiment of the present disclosure the test device and method may be configured to identify a person as being pregnant if the level of hCG in the sample is above a threshold level, wherein the threshold level is varied dependent on the level of LH in the sample. In particular, the threshold level for hCG may be increased or decreased in accordance with a level of LH determined to be present in the sample. The increase may be linear, stepped, logarithmic or otherwise. The threshold level for hCG may be varied continuously or discretely based on the level of LH present. Where the threshold level is varied discretely, the variation may be between e.g., two distinct threshold levels only, although more than two threshold levels may also be used. While variation of threshold levels in relation to hCG and LH is now described, the same approach may be taken in relation to other analytes used to identify the same or other target conditions in accordance with the present disclosure.

As an example of discrete variation, a particular threshold level for LH (T_{LH}) may be used and, if the level of LH present is < T_{LH}, the threshold level for hCG (T_{hCG}) may be set at a relatively low level (T_{hCG_low}) and, if the level of LH present is ≥ T_{LH}, the threshold level for hCG may be set at a relatively high level (T_{hCG_high}). In this example, if the LH level present is < T_{LH} then pregnancy would be identified only if the hCG level was > T_{hCG_low}. On the other hand, if the level of LH present was ≥ T_{LH} then pregnancy would be identified only if the hCG level was > T_{hCG_high}. This example is represented in tabular form in Table 1 below. By taking the approach described, a highly accurate test may be provided, while allowing for some cross reactivity of hCG with LH.

The difference between T_{hCG_low} and T_{hCG_high} may be at least 5 IU/L, at least 10 IU/L or at least 15 IU/L or otherwise. In one embodiment, T_{LH} may be about 20 IU/L, T_{hCG_low} may be about 1 IU/L and T_{hCG_high} may be about 20 IU/L, for example. The levels for T_{LH and} T_{hCG} may be changed, however, to achieve a desirable balance between producing a sensitive test and reducing the possibility of physiological noise affecting the accuracy of the test. Furthermore, the levels may be varied depending on changes in diagnostic practices in the medical industry and/or legal and regulatory requirements.

**Table 1**

| **LH level** | **hCG level** | **Pregnancy** |
|---|---|---|
| < T_{LH} | > T_{hCG_low} | Yes |
| < T_{LH} | < T_{hCG_low} | No |
| ≥ T_{LH} | > T_{hCG_high} | Yes |
| ≥ T_{LH} | < T_{hCG_high} | No |

Nonetheless, alternative approaches to identifying pregnancy may be taken in accordance with the present disclosure. For example, in one embodiment, the test device and method may be configured such that pregnancy is identified based on a determination that hCG is above a threshold hCG level in the sample and LH is below a threshold LH level in the sample, without any variation of either threshold level. In an alternative embodiment, the apparatus may be configured to determine that it is not possible to identify pregnancy, regardless of the level of hCG present in the sample, due to the level of LH being above a threshold LH level.

As indicated further above, the test device and method may be configured for identifying more than one target condition, e.g. first and second target conditions, which target conditions may capable of being present in the body at the same time, or which target conditions may be mutually exclusive. Accordingly, while in the example above identification of LH is effectively used to identify a non-target condition (ovulation period or menopause) for the sole purpose of the accurate identification of a target condition (pregnancy), in other embodiments, a second condition may also be treated as a target condition and therefore information about the second condition may be presented to the user. Identification of a second target condition may be based on identification of the second analyte only, or it again may be based on identification on both the first and second analytes.

Staying with a pregnancy example, the first analyte may be hCG and the first target condition may be pregnancy, and the second analyte may be LH and the second target condition may be the ovulation phase in a menstrual cycle.

As discussed, an LH surge occurs approximately 24-48 hours before ovulation and LH remains elevated during ovulation. Accordingly, while identification of LH can be used to determine if hCG levels are likely to be elevated in the person under test, it may also be used to identify if the person is in (or close to) the ovulation phase of their menstrual cycle, a phase around which sexual intercourse is most likely to result in pregnancy.

Following from this, in one embodiment of the present disclosure the test device and method may be configured, for example, to (i) identify a person as being pregnant if the level of hCG in the sample is above a threshold level, wherein the hCG threshold level is varied dependent on the level of LH in the sample, and (ii) if the person is not identified as being pregnant, identify the person as being in the ovulation phase if the level of LH in the sample is above a threshold level. The arrangement may be similar to the preceding example except, when the level of LH is above T_{LH} and the level of hCG is below T_{hCG_high}, the person providing the sample may be identified not only as being not pregnant, but identified as being in the ovulation phase of their menstrual cycle. This example is represented in tabular form in Table 2 below.

**Table 2**

| **LH level** | **hCG level** | **Ovulation phase** | **Pregnancy** |
|---|---|---|---|
| < T_{LH} | > T_{hCG_low} | No | Yes |
| < T_{LH} | < T_{hCG_low} | No | No |
| ≥ T_{LH} | > T_{hCG_high} | No | Yes |
| ≥ T_{LH} | < T_{hCG_high} | Yes | No |

Thus, the test device and method of the present disclosure may provide means for identifying both pregnancy and the ovulation phase. Since the test device may be a unitary device, e.g. a hand-held device, the device may therefore be used easily at home, when a woman is trying to conceive, or contrarily as a contraceptive device when they are trying not to conceive, and also when they are pregnant. The device may therefore provide a combined ovulation prediction kit (OPK) and home pregnancy test (HPT).

In embodiments of the present disclosure, the test device and method may utilise one or more lateral flow test strips, which may employ principles of immunochromatography, for example. The test device and method may be configured to identify each of the analytes using respective test strips. Alternatively, a single test strip may be used to identify more than one or all of the analytes. In the latter case, the apparatus may provide cost savings since a single test strip may be used to test for multiple conditions e.g. both ovulation and pregnancy, and the test device may be simpler to use than traditional home pregnancy and ovulation test kits.

Follicle Stimulating Hormone (FSH), estradiol and/or progesterone hormones, or their metabolites, may be monitored in place of the LH hormone in some embodiments, which hormones are also present in urine and blood of the general population of women at levels that vary during the menstrual cycle.

The test device and method may employ a reader to identify at least the first and second analytes. The reader may include a photodetector capable of monitoring light reflection or light output at one or more test portions located on the one or more test strips, for example. At least where multiple analytes are to be identified in a single test strip, the test strip may employ fluorescent structures, e.g. quantum dots, as labels for the respective analytes, which structures may be configured to fluoresce at different wavelengths such that the presence of the different structures can be monitored independently, e.g. by a multi-wavelength photodetector, or by separate photodetectors tuned to different wavelengths.

The reader may comprise a processor for processing signals from the one or more photodetectors and identifying the one or more target analytes from the signals. The processor may be connected to a display for presenting information about identification of target conditions to the user.

In an aspect of the present disclosure, there is provided a lateral flow test strip adapted to identify both a first analyte that provides an indicator of pregnancy and a second analyte that provides an indicator of the ovulation phase in a menstrual cycle.

In another aspect of the present disclosure, there is provided a lateral flow test strip adapted to identify both human chorionic gonadotropin (hCG) and luteinizing hormone (LH).

In an example related to the present disclosure, there is provided a lateral flow test strip for identifying in a biological sample a first analyte that provides an indicator of pregnancy and a second analyte that provides an indicator of an ovulation phase in a menstrual cycle, the test strip comprising:
a label-holding portion including a plurality of first and second label-conjugated antibodies, the first label-conjugated antibodies each comprising a first fluorescent structure and configured to bind to molecules of the first analyte in the biological sample to form labelled first analyte complexes, and the second label-conjugated antibodies each comprising a second fluorescent structure and configured to bind to molecules of the second analyte in the biological sample to form labelled second analyte complexes; and
a test portion configured to immobilize both the labelled first analyte complexes and the labelled second analyte complexes;
wherein, upon excitation by light, the first fluorescent structures are configured to fluoresce at a first wavelength and the second fluorescent structures are configured to fluoresce at a second wavelength different from the first wavelength.

In another example related to the present disclosure, there is provided a reader for identifying in a biological sample a first analyte that provides an indicator of pregnancy and a second analyte that provides an indicator of the ovulation phase in a menstrual cycle, the reader comprising:
a housing adapted to at least partially receive a lateral flow test strip and position a test portion of the test strip adjacent one or more light sources and one or more photodetectors, and
a processor connected to the one or more photodetectors,
wherein upon illumination of the test portion of the test strip by the one or more light sources, the processor is configured to receive signals from the one or more photodetectors indicative of (i) an intensity of light of a first wavelength emitted from a plurality of first fluorescent structures at the test portion; and (ii) an intensity of light of a second wavelength emitted from a plurality of second fluorescent structures at the test portion, wherein the second wavelength is different from the first wavelength.

In the preceding two examples, the first analyte may be human chorionic gonadotropin (hCG) and the second analyte may be luteinizing hormone (LH).

The reader may comprise a display. The display may be connected to the processor.

The test strip and the reader may be adapted to identify, based on the signals from the one or more photodectectors, if a woman providing the biological sample is pregnant or not pregnant, or if the woman is pregnant or ovulating or neither pregnant nor ovulating. To this extent the reader may adapted to identify pregnancy and/or ovulation in accordance with discussions made with respect to preceding aspects of the present disclosure, and the reader may display the results of identification to the user via the display.

In another example related to the present disclosure, rather than pregnancy, the target condition may be prior subjection to myocardial infarction ("heart attack"), in which case the first analyte may be Troponin T and the second analyte may be an analyte providing a marker of renal failure such as creatinine. A common marker analyte used for detecting if a person has suffered myocardial infarction (MI) is Troponin T (TNT). TNT is a protein found almost exclusively in heart muscle and is involved in the contraction of the muscle. If a person has been the subject of MI, their heart muscle is oxygen deprived and some of the cells in the heart cannot maintain electrochemical balance and therefore the myocytes can lyse. Myocyte lysis results in a rise in TNT in the blood providing evidence that a heart attack has occurred. TNT levels rise over hours to days and remain high for about 1 to 2 weeks after MI.

Normally, TNT is cleared by the kidneys. However, if a person has renal failure, it is possible to have a background level of TNT that has not been cleared. Renal failure may result in higher levels of TNT and thus lead to false positive indications in diagnostic assays that analyse TNT levels only. Serious complications might arise from such a mistake, e.g. through unnecessarily providing thrombolysis to a patient as a means of treating MI and exposing them to subsequent risks such as intracranial haemorrhage, for example.

An example marker analyte for renal failure is creatinine. Creatinine is filtered out of the blood by the kidneys and therefore, if renal function is inhibited, the level of creatinine in blood rises. A creatinine clearance (CrCL) test may be used to assess renal function.

Following from this, in an example related to the present disclosure the test device may be configured to identify a person as having suffered MI if the level of TNT identified in the sample is above a threshold level, wherein the threshold level is varied dependent on the level of creatinine identified in the sample.

Nonetheless, alternative approaches may be taken. For example, in one embodiment, the test device may be configured such that a person can be deemed as having suffered MI based on a determination that TNT is above a threshold TNT level in the sample and creatinine is below a threshold creatinine level in the sample. In an alternative embodiment, the test device may be configured to determine that it is not possible to identify that a person has suffered MI, regardless of the level of TNT present in the sample, due to the level of creatinine found in the sample.

In examples related to the present disclosure, the second or any successive analyte identified may be an analyte that is normally present in the biological sample under test. The analyte may therefore be used as a form of sample control, identifying in particular whether enough of the sample is present to enable identification of the first target condition by virtue of identification of at least the first analyte. For example, the biological sample may be nasal mucus, the target condition may be influenza such as influenza A, the first analyte may be an influenza antigen such as an influenza viral nucleoprotein antigen and the second analyte may be mucin protein (MUC5A) which is normally present in nasal mucus.

Following from this, the test device and method may be configured such that:
a person can be identified as having influenza based on a determination that the influenza antigen is equal to or above a threshold antigen level (T_{Flu}) in the sample, regardless of the level of MUC5A protein in the sample;
a person can be identified as not having influenza based on a determination that the influenza antigen is below a threshold antigen level (T_{Flu}) in the sample and the level of MUC5A protein in the sample is equal to or above a threshold level (T_{MUC5A}); and/or
the apparatus can indicate that identification of influenza in the person is not possible or unknown, due to the sample being inadequate in size, based on a determination that the influenza antigen is below a threshold antigen level (T_{Flu}) in the sample and the MUC5A protein is below a threshold MUC5A level in the sample (T_{MUC5A}).

When each of these approaches is combined, the test device and method may be configured to identify influenza in accordance with Table 3 below.

**Table 3**

| **Influenza antigen level** | **MUC5A level** | **Influenza** |
|---|---|---|
| ≥ T_{Flu} | Any | Yes |
| < T_{Flu} | ≥ T_{MUC5A} | No |
| < T_{Flu} | < T_{MUC5A} | Unknown |

The test device method may passively or actively identify target conditions. Passive identification of the first target condition, for example, may involve the test device displaying to the user separate information about the identification of the first and second analytes, whereupon the user can identify pregnancy, in accordance with the preceding discussions, based on their own interpretation of the separately displayed information about the first and second analytes. For example, the one or more test portions may display or not display a symbol, or display different symbols, dependent on the identification of the respective analytes. Active identification may involve the test device receiving data representative of the identification of the first and second analytes, processing this data to identify the target condition, and displaying information identifying the target condition(s) to the user. For example, the test device may display or not display a symbol, or display different symbols, dependent on the identification of the target condition.

The test device may comprise a processor, e.g. a computer processor, to process data relating to identification of the first analyte, the second analyte and/or the target condition. The processor may determine if measured levels of the first and/or second analytes are above, equal to, or below a threshold level and the processor may vary ("tune") threshold levels depending on the measured levels of the first and/or second analytes. The processor may be connected to a display device, e.g. a digital display such an LCD or LED display, to display the information about the identification of at least the target conditions.

The one or more test portions may take a variety of different forms suitable for testing the respective analytes. For example, where the analytes hCG and LH are under test or otherwise, the one or more test portions may be comprised in one or more lateral flow means and may employ principals of immunochromatography (rapid flow tests) or otherwise.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Brief Description of Drawings

Embodiments of the present disclosure will now be described by way of specific example with reference to the accompanying drawings, in which:
Fig. 1 shows an oblique view of a test device according to a first embodiment of the present disclosure;
Fig. 2 shows a top view of a test strip used in the test device of Fig. 1;
Fig. 3 shows a cross-sectional view of the test device of Fig. 1 along line A--A of Fig. 1;
Fig. 4 shows a schematic representation of reading apparatus used in the test device of Fig. 1;
Fig. 5a shows a graph of example concentrations of hCG and LH in urine during the menstrual cycle; and Fig. 5b shows a graph of example concentrations of hCG and LH in urine during a life cycle;
Fig. 6 shows a flow chart indicating processing steps of a test device according to a second embodiment of the present disclosure.
Fig. 7 shows an oblique view of a test device according to a ralated example
Fig. 8 shows a top view of test strips used in the test device of Fig. 7;
Fig. 9 shows a schematic representation of reading apparatus used in the test device of Fig. 7
Fig. 10 shows a representation of a test device according to another related example.
Figs. 11a and 11b show opposing side views of the device of Fig. 10, and Fig. 11c shows an end view of the device of Fig. 10; and
Fig. 12 shows representations of different arrangements of darkened stripes that give rise to various identification states of the device of Fig. 10;
Fig. 13a shows distribution of urine samples by type, the samples being obtained from a plurality of women over multiple menstrual cycles, in relation to an experimental example of the present disclosure;
Fig. 13b represents the age distribution of the women providing the samples in the experimental example;
Figs. 14a and 14b provide graphs showing percentages of false positive and false negative results, respectively, predicted for samples in the experimental example, for different hCG threshold levels and with and without the application of LH filtering based on an LH threshold level of about 20 IU/L; and
Fig. 15 provides a graph of inaccuracy rate based on the data of Figs. 14a and 14b.

### Description of Embodiments

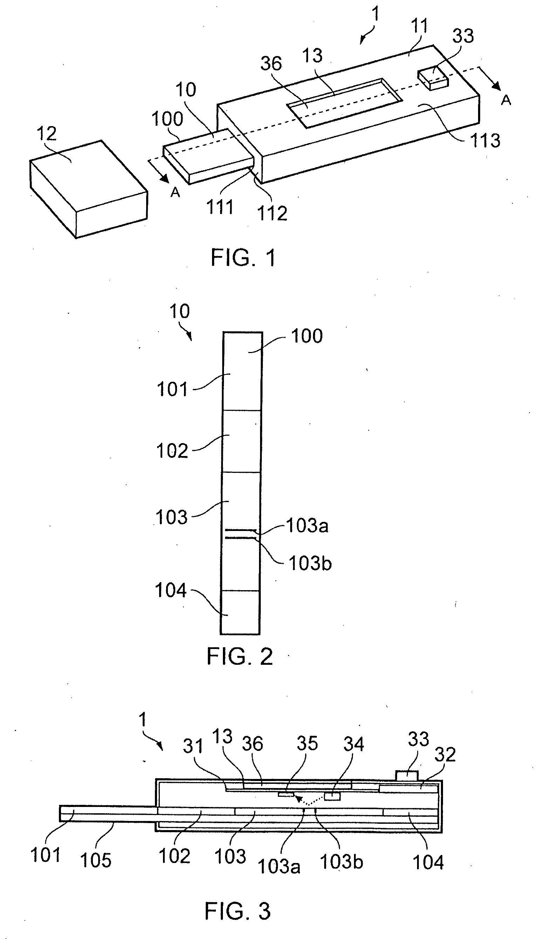

Apparatus, in particular a test device 1, for identifying a target condition in a body according to a first embodiment of the present disclosure is shown in Fig. 1. The test device 1 is configured to test for pregnancy in a woman following receipt of a urine sample from the woman.

The test device 1 includes an elongate lateral flow test strip 10 and a casing 11. The test strip 10 is partially housed in the casing 11 with a sampling end 100 of the test strip 10 protruding from an opening 111 in an end surface 112 of the casing 11, allowing urine sample to be received directly thereon. The sampling end 100 of the test strip 10 is coverable by a cap 12. The test device 1 also includes an LCD display 36 visible through an opening 13 in a top surface 1 1 3 of the casing 11 for displaying results of testing.

The test device 1 is a hand-held device configured to identify pregnancy by identifying amounts (levels) of both hCG and LH hormone in the urine sample. As discussed above, hCG is an indicator (a marker) of pregnancy. However, the amount of hCG present in a woman's urine sample can be elevated outside of pregnancy, particularly during the ovulation phase of a woman's cycle, and around the menopause. At these times, LH is also elevated, and LH can therefore provide a marker for ovulation and menopause. However, particularly in relation to the current embodiment, LH can also provide a marker for identifying when hCG may be at higher background levels in the urine (or blood) of the person under test.

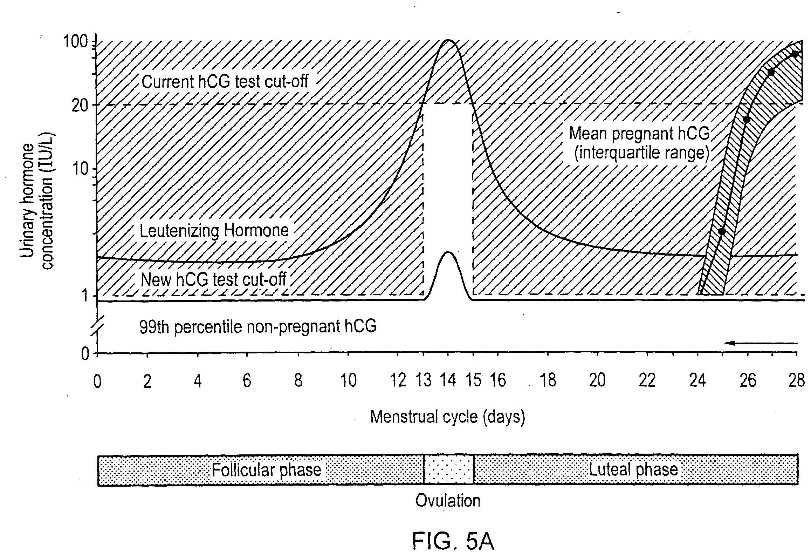

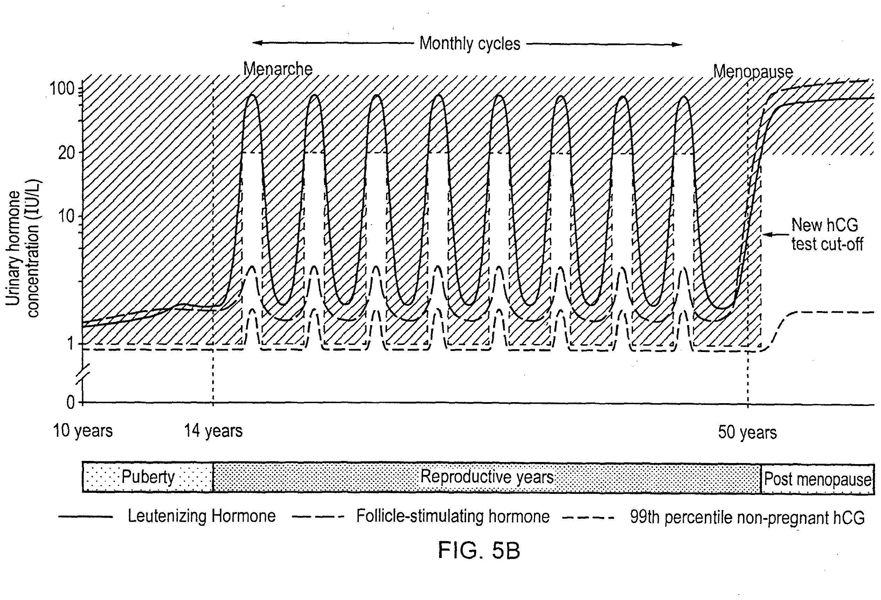

Exemplary changes in hCG and LH throughout the menstrual cycle, and during the first few days of pregnancy is represent graphically in Fig. 5a. These changes, and changes during the menopausal period in an entire life cycle, are also represented graphically in Fig. 5b. As can be seen, the 99^{th} percentile level of hCG in urine of non-pregnant women (i.e. the normal background level of hCG) remains below 1 IU/L throughout the menstrual cycle except during the period around ovulation and menopause, where it increases above 1 IU/L. Accordingly, tests configured to identify pregnancy based simply on an hCG threshold level of 1 IU/L, for example, can provide false positive results during the ovulation phase of the menstrual cycle, and during menopause. For this reason, traditional pregnancy tests set an hCG threshold level of much greater than 1 IU/L, e.g. at about 20 IU/L, which is represented in Fig. 5a by the broken line described as "Current hCG test cut-off". As can be seen from the line at the right side of the graph, however, during the first two or three days of pregnancy the level of hCG in urine remains significantly below the current hCG test cut-offline. Accordingly, traditional pregnancy tests will commonly provide false negatives results during the first few days of pregnancy.

To allow earlier identification of pregnancy, the test device of the present embodiment is configured to set the hCG threshold level for identifying pregnancy at a higher level, e.g. at the current hCG test cut-off level, only when the person under test is at or near the ovulation phase of the menstrual cycle, or in menopause. The change in the hCG threshold level is represented in Figs. 5a and 5b by the broken line described as "New hCG test cut-off". During the rest of the menstrual cycle and during menopause, the test device sets the hCG threshold level at a much lower level, 1 IU/L. To determine when the person under test is at or near the ovulation phase of their menstrual cycle or in menopause, the test device also identifies the levels of LH in the sample. As can be seen from the lines in the graphs representing changes in Leutenizing Hormone (LH) during the menstrual cycle and in menopause, LH surges in the period just before ovulation occurs, and remains elevated during ovulation and is elevated during menopause. In this embodiment, the LH threshold level at which the device sets the higher hCG threshold level is 20 IU/L.

Thus, the device 1 is configured to adjust the manner in which it identifies pregnancy based on identification of different levels of hCG and LH in the sample. The features of the device that enable this to be achieved in the present embodiment are discussed in more detail below.

Referring to Figs. 2 and 3, the test strip 10 is a lateral flow test strip including different zones arranged sequentially along the length of the strip, including a sample receiving zone 101 at the sampling end 100, a label-holding zone 102, a test zone 103, and a sink 104. The zones 101-104 comprise chemically treated nitrocellulose, located on a waterproof substrate 105. The arrangement of the test zones 101-104 and substrate 105 is such that the urine sample, when directed onto the sample receiving zone 101, is absorbed into the sampling receiving zone 101 and travels under capillary action sequentially through the sample receiving zone 101, the label-holding zone 102, and the test zone 103 and accumulates finally at the sink 104.

The label-holding zone 102 comprises three types of label-conjugated antibodies in this embodiment. Two of the label-conjugated antibodies are designed to bind, respectively, with the hCG and LH hormone molecules to form complexes. The third label-conjugated antibody is designed for use as a control. The mix of the sample, the different LH and hCG complexes and the control label-conjugated antibody can travel to the test zone 103 and contact a test stripe 103a that contains immobilized compounds capable of binding the LH and hCG labelled complexes. When a sufficient amount of sample is present, the mix will continue through the text zone to contact a control stripe 103b capable of binding the control label-conjugated antibody.

In this embodiment, the three label-conjugated antibodies are labelled with different types of fluorescent quantum dots (QDs), configured to fluoresce at a different specific emission peak wavelengths following UV light excitation (e.g. 525, 625 and 800nm, respectively). Accordingly, by illuminating the stripes 103a, 103b with UV light, the presence of the QD labels will result in a detectable light emission with different emission peaks. The intensity of the light emission (the size of the peaks) is indicative of the number of labelled complexes/antibodies bound to the stripes, which is in turn indicative of the prevalence of hCG and LH hormone in the sample and the amount of the sample that has reached the control stripe. As such, one or more wavelength sensitive photodetectors, forming part of a reader, can be used to identify the amounts of hCG and LH hormone in the sample through monitoring of the test stripe 103a. The one or more photodetectors can also be used to determine, through monitoring of the control stripe 103b, that a sufficient amount of sample has travelled through the test stripe 103a to the control stripe 103b and that binding of the labelled complexes has been successful.

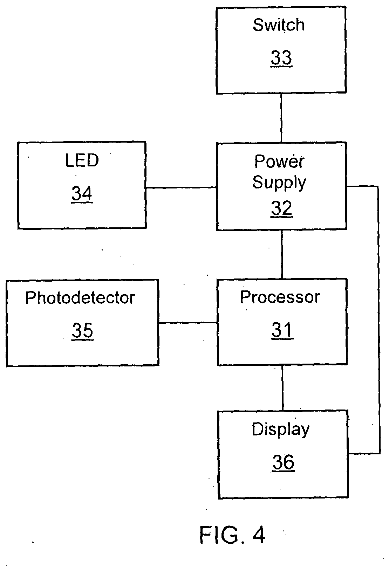

Referring to Figs. 3 and 4, reading apparatus of the test device 1 is now described in more detail. The reading apparatus includes a printed circuit board having a processor 31, a power supply (battery) 32, a switch 33, a UV LED 34, a multi-wavelength photodetector 35 and the display 36. The LED 34 is configured to emit light in the UV spectrum (at about 300 to 400 nm), that is incident on the stripes 103a, 103b to cause excitation of the quantum dot labels located thereon. The multi-wavelength photodetector 35 in combination with the processor 31 is configured to detect the different intensities of light emitted from the quantum dots at each of the three distinct wavelengths.

In use, the cap 12 is removed from sampling end 100 of the test strip and a urine sample is directed onto the sample receiving zone 101. The cap 12 can be replaced and, after approximately 1 or 2 minutes, giving sufficient time for the lateral flow process to take place, the switch 33 can be depressed, causing flow of electricity from the power supply 32 to the LED 34, resulting in emission of UV light from the LED 34 that is incident on the stripes 103a, 103b of the test strip 10. The UV light results in excitation of any or all of the three types of quantum dots that may be immobilized as part of the respective labelled complexes at the stripes 103a, 103b, causing light emission at respective wavelength peaks. In combination with the multi-wavelength photodetector 35, the processor 31 is configured to determine the size of the emission peaks and identify from this (a) if the sample mix has arrived at the control stripe 103b and labelling has been effective, and if yes, identify (b) an amount of hCG present in the sample, and (c) an amount of LH present in the sample.

While a manual switch 33 is described above, in alternative embodiments, switching may be automated. For example, switching may be configured to occur upon replacement of the cap 12 onto the casing 11 or due to fluid activation, as the sample travels through a fluid-activated switch that may be provided in the device.

If it is identified there is insufficient amount of sample, the processor 31 is configured to cause the display 36 to present the words INVALID TEST.

If it is identified there is sufficient amount of sample, the processor 31 is configured to identify the levels of hCG and LH in the sample. Specifically, the processor in this embodiment is configured to determine if the level of LH is equal to or greater than an LH threshold level (T_{LH}) of 20 IU/L. If the level of LH present is less than the threshold level, the processor sets an hCG threshold level (T_{hCG_low}) for identifying pregnancy of 1 IU/L, i.e. it identifies pregnancy only if the level of hCG is greater than 1 IU/L. On the other hand, if the level of LH is greater than or equal to the LH threshold level (T_{LH}) of 20 IU/L, the processor sets a higher hCG threshold level (T_{hCG_high}) for identifying pregnancy of 20 IU/L, i.e. it identifies pregnancy only if the level of hCG is greater than 20 IU/L. The approach is represented in the graph of Figs. 5a and 5b discussed above, and is also represented in Table 4 below.

**Table 4**

| **LH level** | **hCG level** | **Pregnancy** | **Display** |
|---|---|---|---|
| < 20 | > 1 | Yes | PREGNANT |
| < 20 | < 1 | No | NOT PREGNANT |
| ≥ 20 | > 20 | Yes | PREGNANT |
| ≥ 20 | < 20 | No | NOT PREGNANT |

The LED may be carefully calibrated to ensure that the light emission from the LED is consistent from one device to the next, ensuring that a degree of excitation of the quantum dots is consistent. Additionally or alternatively, a calibration mechanism may be integrated into the device. A known quantity of quantum dots, configured to fluoresce at yet another wavelength, may be immobilized on the strip, e.g. at the test stripe. Depending on the intensity of the fluorescence detected from the known quantity of quantum dots, the processor may adjust its interpretation of the light emission from quantum dots that label the LH and hCG analytes. Additionally or alternatively, multiple LEDs may be used to excite the quantum dots with a view to suppressing the overall effect of any rogue LEDs.

If the processor 31 determines that the level of hCG is at or above the relevant hCG threshold level, the processor 31 causes the display 36 to present the words PREGNANT. If the processor 31 determines that the level of hCG is below the relevant hCG threshold level, the processor 31 causes the display 36 to present the words NOT PREGNANT.

In a second embodiment of a device according to the present disclosure, substantially the same device as described above with respect to the first embodiment is provided, but the device is configured to identify both pregnancy and ovulation phase in a woman's cycle. The difference between the devices of these two embodiments resides in the manner in which the processor 31 is configured to process information about the levels of LH and hCG in the sample and display information via the display 36.

As discussed, an LH surge occurs before ovulation and LH remains elevated during ovulation, and hCG levels are elevated during this period. Accordingly, in the first embodiment, identification of LH is used to determine if hCG levels are likely to be elevated in the person under test. However, in the second embodiment, identification of LH is used also to identify if the person is in (or close to) the ovulation phase of their menstrual cycle, a phase around which sexual intercourse is most likely to result in pregnancy.

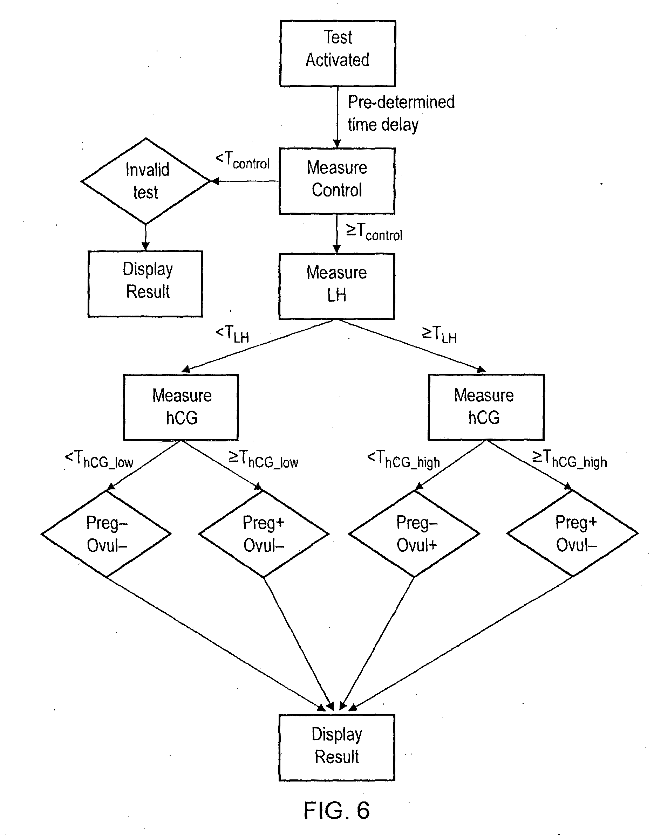

In this embodiment, if the level of LH is greater than or equal to the LH threshold level (T_{LH}), again the processor sets the higher hCG threshold level (T_{hCG_high}) for identifying pregnancy. However, if the level of hCG is lower than T_{hCG_high}, the processor identifies the ovulation phase of the woman's cycle and this information is conveyed to the user via the display 36. The same threshold level values for LH and hCG are used as those used in the preceding embodiment, although alternative values may be used. The approach is represented in Table 5 below. A flow-chart representing various processing steps taken by the processor in this second embodiment is also shown in Fig. 6.

**Table 5**

| **LH level** | **hCG level** | **Ovulation phase** | **Pregnancy** | **Display** |
|---|---|---|---|---|
| < 20 | > 1 | No | Yes | PREGNANT |
| < 20 | < 1 | No | No | NOT PREGNANT |
| | | | | NOT OVULATING |
| ≥ 20 | > 20 | No | Yes | PREGNANT |
| ≥ 20 | < 20 | Yes | No | OVULATING |

Thus, the device in this embodiment is configured to identify both pregnancy and ovulation. Since the device is a hand-held device, the device may be used at home, both while a woman is trying conceive (or contrarily as a contraceptive device), and also when they are pregnant. The device provides a combined ovulation prediction kit (OPK) and home pregnancy test (HPT).

The device is configured to allow removal of a used test strip from the casing 10, via the opening 111, and allow placement of a new test strip into the casing 10, via the same opening 111. Each time the strip is replaced, an identically configured test strip can be used, regardless of whether a woman is seeking to test for one or both of ovulation or pregnancy. In alternative embodiments, the device may be entirely a single-use device.

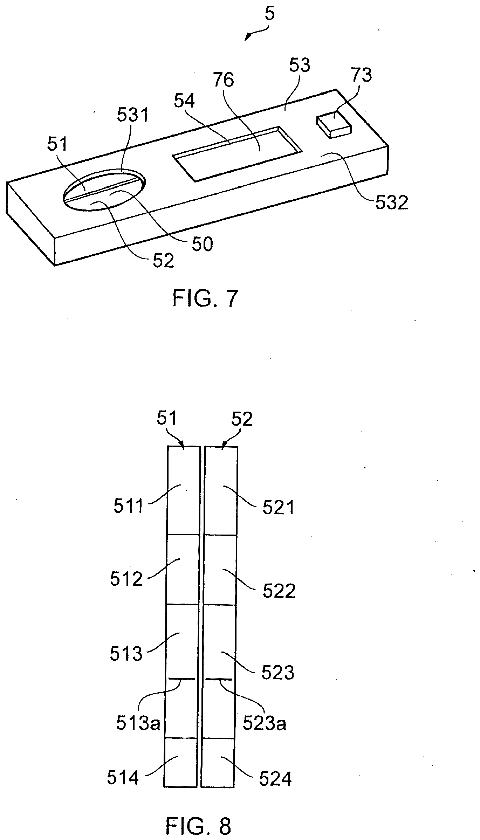

A test device 5, for identifying a target condition in a body according to a related example is represented in Fig. 7. The test device 5 is configured to test for prior subjection to myocardial infarction (MI or "heart attack").

The test device 5 includes two elongate lateral flow test strip 51, 52 and a casing 53. The test strips 51, 52 are each substantially housed in the casing 53 with a sampling end 50 of each of the test strips 51, 52 exposed through an opening 531 in a top surface 532 of the casing 53 allowing a blood sample, e.g. produced by a finger prick or applied via a pipette or otherwise, to be received directly thereon. Buffer solution can be added to increase the fluidity of the blood sample and assist lateral flow through the test strips 51, 52. The test device 5 also includes an LCD display 76 visible through an opening 54 in the top surface 532 of the casing 53 for displaying results of testing.

The test device 5 is a single-use hand-held device configured to identify prior subjection to myocardial infarction (MI) in a patient by identifying the amounts of both Troponin T (TNT) and creatinine in a blood sample from the patient. Levels of Troponin T in a urine sample provide an indication of whether or not a patient has suffered MI. However, the amount of Troponin T in the sample can be affected by the patient's ability to clear TNT from their system, meaning that background level of TNT may be higher in those who have renal dysfunction. The amount of creatinine in a urine sample is indicative of renal function. Following from this, the device 5 is configured to adjust the manner in which it identifies prior subjection to MI based on identification of different levels of TNT and creatinine in the sample. The manner in which this is achieved is discussed in more detail below.

Each of the test strips 51, 52 is a lateral flow test strip including zones arranged sequentially along its length, including a sample receiving zone 511, 521 at the sampling end 50, a label-holding zone 512, 522, a test zone 513, 523, and a sink 514, 524. Each of the test strips 51, 52 is therefore configured in a similar manner, and each works under similar principles, to the test strip 10 described above with respect to Figs. 1 to 5b. However, in this embodiment, a single target analyte only is identified by each test strip respectively, and thus two strips are used (a TNT strip 51 and a creatinine strip 52). Furthermore, the test strips 51, 52 employ dye molecules as labels, rather than quantum dots, and they include no control stripes (although control strips may be used in alternative arrangements).

In more detail, at the label-holding zone 512 of the TNT test strip 51, label-conjugated antibodies are provided that bind with TNT antigens in the sample to form complexes. The mix of the sample and the labelled TNT complexes can travel to the test zone 513 and contact a test stripe 513a that contains immobilized compounds capable of binding the labelled TNT complexes.

Similarly, at the label-holding zone 522 of the creatinine test strip 52, label-conjugated antibodies are provided that bind with creatinine antigens in the sample to form complexes. The mix of the sample and the labelled creatinine complexes can travel to the test zone 523 and contact a test stripe 523a that contains immobilized compounds capable of binding the labelled creatinine complexes.

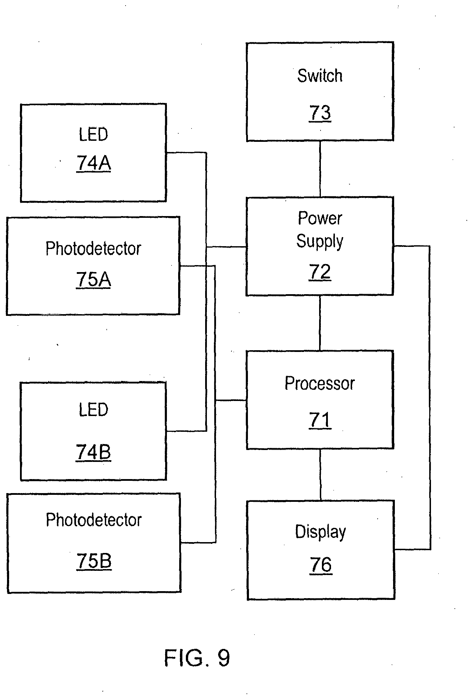

Referring to Fig. 9, reading apparatus of the test device 5 is now described in more detail. The reading apparatus includes a printed circuit board having a processor 71, a power supply (battery) 72, a switch 73, first and second LEDs 74a, 74b, and first and second photodetectors 75a, 75b and the display 76. The first LED 74a is configured to emit light that is incident on the test stripe 513a of the TNT test strip 51 and the first photodetector 75a is configured to monitor the amount of light reflected from the test stripe 513a. Similarly, the second LED 74b is configured to emit light that is incident on the test stripe 513b of the creatinine test strip 52 and the second photodetector 75b is configured to monitor the amount of light reflected from the test stripe 523a. The amount of light reflected off the stripes is dependent on the number of dye molecules bound at the stripes and is therefore indicative of the amount of labelled TNT and creatinine complexes bound at the test stripes 513a, 523a of the TNT and creatinine test strips 51, 52, respectively. A partitioning wall is provided between each LED/photodetector combination to avoid light interference.

In combination with both photodetectors 75a, 75b, the processor 71 is configured to identify if the amount of TNT in the sample is greater than a starting TNT threshold of about 40ng/L. However, the processor 71 is configured to increase this TNT threshold to about 100ng/L if it determines that elevated levels of creatinine are present in the sample, particularly if the levels of creatinine are greater than 150-200 mmol/L for example.

If the processor 71 determines that the level of TNT is above the TNT threshold, the processor 71 causes the display 76 to present the words MI POSITIVE. If the processor 71 determines that the level of TNT is below the TNT threshold, the processor 71 causes the display 76 to present the words MI NEGATIVE.

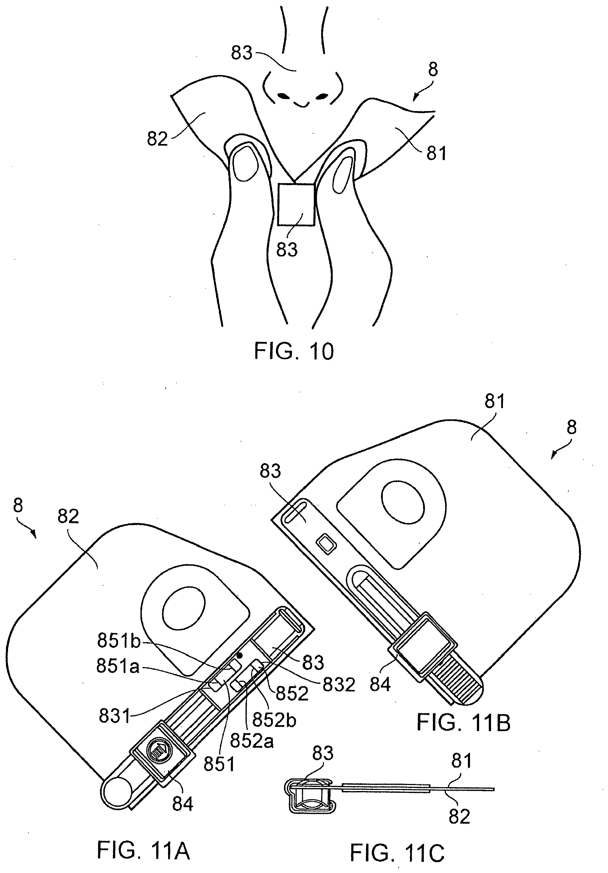

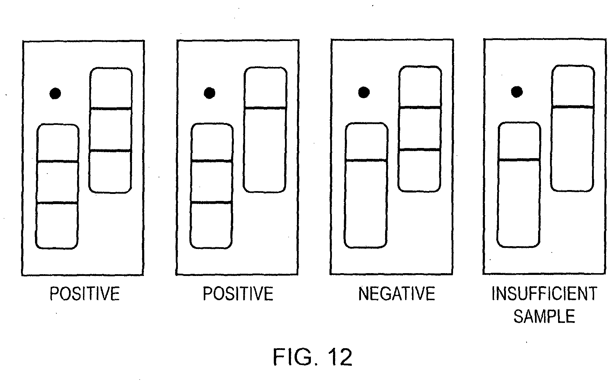

With reference to Figs. 10 to 12, a device 8 according to a another related example is now described. The device 8 may be considered to take, generally, a butterfly shape, due to the inclusion in the device of two wings 81, 82, either side of a housing 83, that are sufficiently pliable to flex around a person's nose 83, permitting the person to deposit a nasal mucus sample in a region between the two wings 81, 82, using a nose blowing technique. Once deposited, a buffer solution can be released from a reservoir in the housing 83 using a slide mechanism 84, increasing the fluidity of the sample and causing the sample to flow under capillary action through the device 8 to two lateral flow test strips 851, 852 located in the housing 83. Respective test portions of the lateral flow test strips 851, 852 are visible through widows 831, 832 in the housing 83. A test stripe 851a, 852a and a control stripe 851b, 852b are located at each of the test portions, as also illustrated in Fig. 12.

Overall, the configuration and function of the device 8 is substantially identical to the device described at page 22, line 28 to page 28, line 3, and depicted in Figs. 7 to 14, of Applicant's PCT Publication No. WO2011/091473A1, the content of which is incorporated herein by reference. However, the presently disclosed device provides two test strips 851, 852 that are configured to identify in combination a single first target condition, influenza A. The first test trip 851 is configured to identify an influenza viral nucleoprotein antigen, which provides a marker of influenza A, and the second test strip 852 is configured to identify a mucin protein (MUC5A), which provides a marker of the size of the nasal mucus sample received by the device.

MUC5A is normally present in nasal mucus. Since buffer solution is used to assist in lateral flow of the sample through the test strips 851, 852, the sample, including MUC5A, can be diluted. If there is too much dilution, there is a risk that insufficient biological sample may reach test stripes 851a, 852a. Accordingly, the size of the sample reaching the test stripe 852a of one of the test strips 852, which has virtually identical lateral flow properties to the other of the test strips 851, can be assessed through identification of dye-labelled MUC5A complexes bound at the test stripe 852a.

The device 8 is configured to allow passive identification of influenza A through visual analysis by the user of test and control stripes 851a, 852a, 851b, 852b of each strip 851, 852. Particularly, identification is achieved through visually checking which of the stripes 851a, 852a, 851b, 852b are darkened shortly after application of nasal mucus and release of the buffer solution. When the test stripes 851a, 852a in particular are darkened, it is an indication that the amount of influenza A and MUC5A dye-labelled molecules immobilized at these test stripes, respectively, exceeds respective predetermined threshold levels.

The device 8 allows a person to be identified as having influenza A based on identification that the amount of the influenza A antigen is equal to or greater than the threshold antigen level (test stripe 851a is darkened), regardless of the level of MUC5A in the sample (test stripe 852a may or may not be darkened). The device also allows a person to be identified as not having influenza A based on a determination that the influenza A antigen is below a threshold antigen level (test stripe 851a is not darkened), when the level of MUC5A in the sample is equal to or above a threshold level (test stripe 852a is darkened). Further, the device informs a person that identification of influenza A in the person is not possible, due to the sample being inadequate in size, based on a determination that the influenza A antigen is below a threshold antigen level (test stripe 851a is not darkened), but the level of MUC5A in the sample is below a threshold level (test strip 851b is not darkened). The different arrangements of darkened stripes that give rise to the various identification states described above are represented to the user in pictorial form, as shown in Fig. 12, either on the device itself and/or in an accompanying instruction booklet.

While the device 8 of the above-described related example provides for passive identification of a target condition, in alternative embodiments, a reader may be included in the device 8 that analyses the test stripes 851a, 852a, 851b, 852b using one or more photodetectors and actively identifies the different identification states and presents information about the identification to the user via a digital display for example.

### Experimental Example

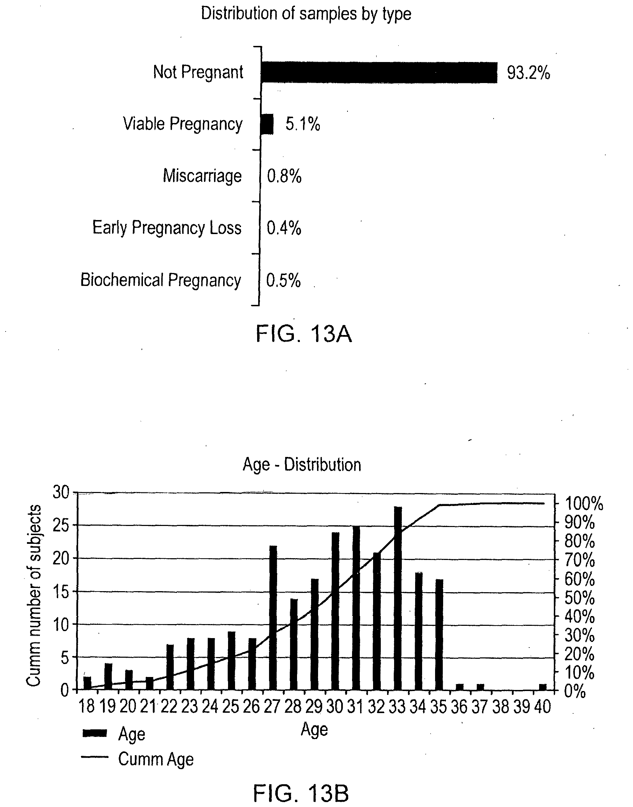

Urine samples were obtained daily from 240 women. The age range of the women was 18 to 40 years and the average age of the women was 29.5 years. The samples were obtained across a cumulative total of 943 different menstrual cycles. Levels of hCG and LH in a total of 11,557 of the samples were measured using the Siemens Immulite 1000 and 2000 platforms. Each sample was ultimately categorised by sample type, based on whether or not the woman providing the sample was subsequently determined to have been pregnant or not pregnant at the time that she had provided the sample. Pregnancy was broken down into different categories including biochemical pregnancy, or pregnancy culminating in early pregnancy loss, miscarriage, or a viable pregnancy. A chart representing the distribution of the samples by type is provided in Fig. 13a and a chart representing the age distribution of the women is provide in Fig. 13b.

Multiple predictions of pregnancy or non-pregnancy were made for each sample by comparing (a) the measured hCG and LH levels of the sample with different combinations of hCG and LH threshold levels, and by comparing (b) the measured hCG level of the sample with different hCG threshold levels only. Approach (a) can be considered to apply LH 'filtering', whereas approach (b) can be considered to apply no such filtering. In (a), for each different combination of hCG and LH threshold levels, a positive result (i.e. pregnancy) was predicted only where the measured level of hCG was above the hCG threshold level and where the measured level of LH was below the LH threshold level. In (b), for each hCG threshold level, a positive result was predicted only where the measured level of hCG was above the hCG threshold level, without regard to the measured LH level. A negative result (i.e. non-pregnancy) was predicted in all other instances. Each predicted result was then compared with the sample type, to determine if the predicted result was a false positive result or a false negative result.

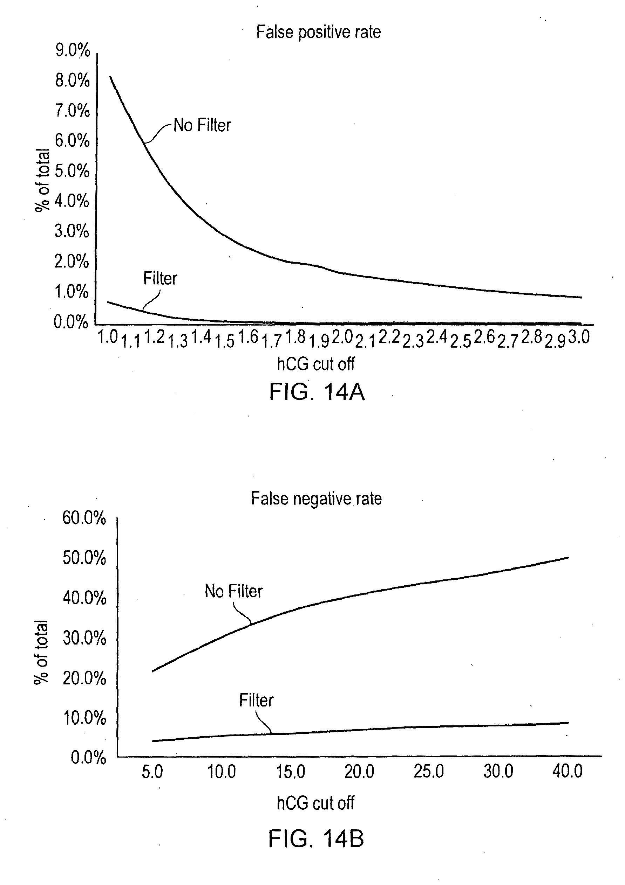

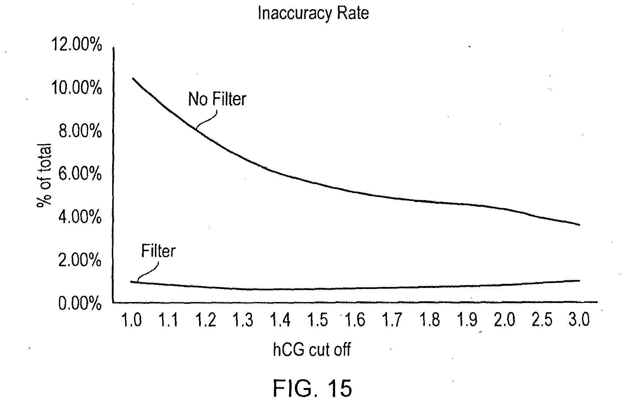

Figs. 14a and 14b provide graphs showing the percentage of false positive and false negative results, respectively, which were predicted for different hCG threshold levels, (a) when LH filtering was applied, in particular an LH threshold level of about 20 IU/L in this instance and (b) when no LH filtering was applied. An inaccuracy rate (i.e. percentage at which any false result was predicted) is also represented graphically in Fig. 15. As can be seen, an approximately 10 fold improvement in accuracy of testing is achieved by applying filtering based on an LH threshold level of about 20 IU/L. Benefits were also seen at various other LH thresholds (e.g., at LH levels between 10 and 30 IU/L, 15 and 25 IU/L and 18 and 22 IU/L, etc).

The experimental example indicates not only that greater testing accuracy can be achieved by applying filtering based on measured and threshold LH levels, but that identification of pregnancy at an accuracy level equal to or exceeding current standards can be achieved based on much lower levels of measured hCG. This in turn means that earlier identification of pregnancy may be made. For example, whereas the accuracy of current tests (which measure hCG only and determine pregnancy based on hCG measurements > 20 IU/L only) may achieve an acceptable positive test accuracy at an average of about 3 days from implantation, if optimum LH filtering is applied to the sample data acquired in this example, it has been determined that a corresponding degree of positive test accuracy can be achieved as early as about 0.5 days from implantation. This provides an improvement in early-testing capability following implantation of about 2.5 days

It will be appreciated by persons skilled in the art that numerous variations and/or modifications may be made to the above-described embodiments, without departing from the broad general scope of the present disclosure. For example, while various threshold levels for testing hCG and TNT, etc., are provided, alternative threshold levels may be used. For example, the levels may be changed to achieve a more desirable balance between producing a sensitive test and eliminating the possibility of physiological noise affecting the accuracy of the test. Furthermore, the levels may be varied depending on changes in diagnostic practices in the medical industry or legal requirements. While embodiments of test devices for receiving urine and blood samples are described, the test devices may be adapted for receiving other types of samples. Furthermore, while embodiments of test devices that employ lateral flow test strips are described, other assays may be used, such as microfluidic devices including lab-on-a-chip (LOC) devices and other types of immunoassays and nucleic acid assays, for example. The present embodiments are, therefore, to be considered in all respects as illustrative and not restrictive.

## Claims

1. A test device (1) for identifying at least pregnancy in a human or animal body, the test device comprising:
one or more test portions (10) for identifying:
a level of a first analyte in a biological sample from the body, the first analyte being human chorionic gonadotropin (hCG); and
a level of a second analyte in the biological sample, the second analyte being luteinizing hormone (LH);
wherein the test device (1) is configured to identify pregnancy in the body if the identified level of hCG is above an hCG threshold level, wherein the test device (1) varies the hCG threshold level depending on the level of LH identified in the sample.

2. The test device (1) of claim 1, wherein the test device is configured to continuously vary the hCG threshold level depending on the level of LH identified in the sample.

3. The test device (1) of claim 1, wherein the test device is configured to discretely vary the hCG threshold level depending on the level of LH identified in the sample.

4. The test device (1) of claim 3, wherein the test device sets the hCG threshold level to identify pregnancy at a first hCG threshold level if the level of LH identified in the sample is below an LH threshold level, and the test device (1) sets the hCG threshold level to identify pregnancy at a second hCG threshold level if the level of LH identified in the sample is above an LH threshold level, wherein the first hCG threshold level is lower than the second hCG threshold level.

5. The test device (1) of claim 4, wherein the difference between the first and second hCG threshold levels is at least 5 IU/L or at least 15 IU/L.

6. The test device (1) of claim 3, wherein the hCG threshold level to identify pregnancy is set at one of three or more different hCG threshold levels depending on the level of LH identified in the sample.

7. The test device (1) of any one of the preceding claims, wherein the test device (1) is for identifying pregnancy in the body and identifying ovulation phase in a menstrual cycle in the body,
wherein, if pregnancy is not identified in the body, the test device (1) is configured to identify ovulation phase in a menstrual cycle in the body if the identified level of LH in the sample is above an LH threshold level.

8. The test device (1) of any one of the preceding claims comprising a display (36), wherein the test device is configured to present information about the identification of pregnancy to a user via the display (36).

9. The test device (1) of any one of the preceding claims wherein the test device (1) is a hand-held test device.

10. The test device of any one of the preceding claims comprising one or more lateral flow test strips (10), wherein the one or more test portions are comprised in the one or more lateral flow test strips (10).

11. A method for identifying at least pregnancy in a human or animal body, the method comprising:
identifying a first analyte in a biological sample from the body, the first analyte being hCG;
identifying a second analyte in the biological sample, the second analyte being LH; and
identifying pregnancy in the body if the identified level of hCG in the sample is above an hCG threshold level, the hCG threshold level being varied depending on the level of LH identified in the sample.

12. The method of claim 11, wherein the hCG threshold level is discretely varied depending on the level of the second analyte identified in the sample, and the method comprises:
identifying pregnancy in the body if the identified level of hCG in the sample is above a first hCG threshold level and the identified level of LH in the sample is below an LH threshold level; and
identifying pregnancy in the body if the identified level of hCG in the sample is above a second hCG threshold level and the identified level of the second analyte in the sample is above an LH threshold level,
wherein the first hCG threshold level is lower than the second hCG threshold level.

13. The method of claim 12, wherein the difference between the first and second hCG threshold levels is at least 5 IU/ or at least 15 IU/L.

14. The method of claim 11, wherein the hCG threshold level is discretely varied depending on the level of LH identified in the sample and the hCG threshold level to identify pregnancy is set at one of three or more different hCG threshold levels depending on the level of LH identified in the sample.

15. The method of any one of claims 11 to 14, wherein the method is for identifying pregnancy in the body and identifying ovulation phase in a menstrual cycle in the body, the method further comprising:
if pregnancy is not identified in the body, identifying ovulation phase in a menstrual cycle in the body if the identified level of LH is above an LH threshold level.

## Patentansprüche

1. Testvorrichtung (1) zum Identifizieren von zumindest Schwangerschaft in einem menschlichen oder tierischen Körper, wobei die Testvorrichtung Folgendes umfasst:
einen oder mehrere Testabschnitte (10) zum Identifizieren von:
einem Spiegel eines ersten Analyten in einer biologischen Probe aus dem Körper, wobei der erste Analyt menschliches Choriongonadotropin (hCG) ist; und
einem Spiegel eines zweiten Analyten in der biologischen Probe, wobei der zweite Analyt luteinisierendes Hormon (LH) ist;
wobei die Testvorrichtung (1) konfiguriert ist, eine Schwangerschaft in dem Körper zu identifizieren, wenn der identifizierte Spiegel von hCG über einem hCG-Schwellenspiegel liegt, wobei die Testvorrichtung (1) den hCG-Schwellenspiegel abhängig vom Spiegel des in der Probe identifizierten LH variiert.

2. Testvorrichtung (1) nach Anspruch 1, wobei die Testvorrichtung konfiguriert ist, den hCG-Schwellenspiegel abhängig von dem Spiegel des in der Probe identifizierten LH kontinuierlich zu variieren.

3. Testvorrichtung (1) nach Anspruch 1, wobei die Testvorrichtung konfiguriert ist, den hCG-Schwellenspiegel abhängig von dem Spiegel des in der Probe identifizierten LH diskret zu variieren.

4. Testvorrichtung (1) nach Anspruch 3, wobei die Testvorrichtung den hCG-Schwellenspiegel zum Identifizieren einer Schwangerschaft auf einen ersten hCG-Schwellenspiegel einstellt, wenn der Spiegel des in der Probe identifizierten LH unter einem LH-Schwellenspiegel liegt, und die Testvorrichtung (1) den hCG-Schwellenspiegel zum Identifizieren einer Schwangerschaft auf einen zweiten hCG-Schwellenwert einstellt, wenn der Spiegel des in der Probe identifizierten LH über einem LH-Schwellenspiegel liegt, wobei der erste hCG-Schwellenspiegel niedriger ist als der zweite hCG-Schwellenspiegel.

5. Testvorrichtung (1) nach Anspruch 4, wobei die Differenz zwischen dem ersten und dem zweiten hCG-Schwellenspiegel zumindest 5 IU/l oder zumindest 15 IU/l beträgt.

6. Testvorrichtung (1) nach Anspruch 3, wobei der hCG-Schwellenspiegel zum Identifizieren einer Schwangerschaft abhängig von dem Spiegel des in der Probe identifizierten LH auf einen von drei oder mehr verschiedenen hCG-Schwellenspiegel eingestellt wird.

7. Testvorrichtung (1) nach einem der vorangegangenen Ansprüche, wobei die Testvorrichtung (1) zum Identifizieren einer Schwangerschaft im Körper und zum Identifizieren der Eisprungphase in einem Menstruationszyklus im Körper dient,
wobei die Testvorrichtung (1) konfiguriert ist, wenn keine Schwangerschaft im Körper identifiziert wurde, die Eisprungphase in einem Menstruationszyklus im Körper zu identifizieren, wenn der identifizierte Spiegel von LH in der Probe über einem LH-Schwellenspiegel liegt.

8. Testvorrichtung (1) nach einem der vorangegangenen Ansprüche, umfassend eine Anzeige (36), wobei die Testvorrichtung konfiguriert ist, Informationen über die Identifikation der Schwangerschaft einem Benutzer über die Anzeige (36) anzuzeigen.

9. Testvorrichtung (1) nach einem der vorangegangenen Ansprüche, wobei die Testvorrichtung (1) eine Hand-Testvorrichtung ist.

10. Testvorrichtung nach einem der vorangegangenen Ansprüche, umfassend einen oder mehrere Lateralflussteststreifen (10), wobei der eine oder mehrere der Testabschnitte in dem einen oder mehreren der Lateralflussteststreifen (10) umfasst sind.

11. Verfahren zum Identifizieren von zumindest Schwangerschaft in einem menschlichen oder tierischen Körper, wobei das Verfahren Folgendes umfasst:
Identifizieren eines ersten Analyten in einer biologischen Probe aus dem Körper, wobei der erste Analyt hCG ist;
Identifizieren eines zweiten Analyten in der biologischen Probe, wobei der zweite Analyt LH ist; und
Identifizieren einer Schwangerschaft im Körper, wenn der identifizierte Spiegel von hCG in der Probe über einem hCG-Schwellenspiegel liegt, wobei der hCG-Schwellenspiegel abhängig von dem Spiegel von in der Probe identifiziertem LH variiert.

12. Verfahren nach Anspruch 11, wobei der hCG-Schwellenspiegel abhängig von dem Spiegel des zweiten in der Probe identifizierten Analyten diskret variiert wird und das Verfahren Folgendes umfasst:
Identifizieren einer Schwangerschaft im Körper, wenn der identifizierte Spiegel von hCG in der Probe über einem ersten hCG-Schwellenspiegel liegt und der identifizierte Spiegel von LH in der Probe unter einem LH-Schwellenspiegel liegt; und
Identifizieren einer Schwangerschaft in dem Körper, wenn der identifizierte Spiegel von hCG in der Probe über einem zweiten hCG-Schwellenspiegel liegt und der identifizierte Spiegel des zweiten Analyten in der Probe über einem LH-Schwellenspiegel liegt,
wobei der erste hCG-Schwellenspiegel niedriger ist als der zweite hCG-Schwellenspiegel.

13. Verfahren nach Anspruch 12, wobei die Differenz zwischen dem ersten und dem zweiten hCG-Schwellenspiegel zumindest 5 IU/l oder zumindest 15 UI/l beträgt.

14. Verfahren nach Anspruch 11, wobei der hCG-Schwellenspiegel abhängig von dem Spiegel von in der Probe identifiziertem LH diskret variiert wird und der hCG-Schwellenspiegel zum Identifizieren einer Schwangerschaft abhängig von dem Spiegel des in der Probe identifizierten LH auf einen von drei oder mehr verschiedenen hCG-Schwellenspiegel eingestellt wird.

15. Verfahren nach einem der Ansprüche 11 bis 14, wobei das Verfahren zum Identifizieren einer Schwangerschaft im Körper und zum Identifizieren einer Eisprungphase im Menstruationszyklus in dem Körper dient, wobei das Verfahren ferner Folgendes umfasst:
wenn keine Schwangerschaft im Körper identifiziert wird, Identifizieren einer Eisprungphase in einem Menstruationszyklus in dem Körper, wenn der identifizierte Spiegel von LH über einem LH-Schwellenspiegel liegt.

## Revendications

1. Dispositif de test (1) destiné à identifier au moins une grossesse dans un corps humain ou animal, le dispositif de test comprenant :
une ou plusieurs parties de test (10) pour identifier :
un niveau d'une première substance à analyser dans un échantillon biologique du corps, la première substance à analyser étant la gonadotrophine chorionique humaine (hCG) ; et
un niveau d'une seconde substance à analyser dans l'échantillon biologique, la seconde substance à analyser étant l'hormone lutéinisante (LH) ;
dans lequel le dispositif de test (1) est configuré de manière à identifier une grossesse dans le corps si le niveau identifié de gonadotrophine hCG est supérieur à un niveau de seuil de gonadotrophine hCG, dans lequel le dispositif de test (1) fait varier le niveau de seuil de gonadotrophine hCG en fonction du niveau d'hormone LH identifié dans l'échantillon.

2. Dispositif de test (1) selon la revendication 1, dans lequel le dispositif de test est configuré de manière à faire varier en continu le niveau de seuil de gonadotrophine hCG en fonction du niveau d'hormone LH identifié dans l'échantillon.

3. Dispositif de test (1) selon la revendication 1, dans lequel le dispositif de test est configuré de manière à faire varier discrètement le niveau de seuil de gonadotrophine hCG en fonction du niveau d'hormone LH identifié dans l'échantillon.

4. Dispositif de test (1) selon la revendication 3, dans lequel le dispositif de test définit le niveau de seuil de gonadotrophine hCG pour identifier la grossesse à un premier niveau de seuil de gonadotrophine hCG si le niveau d'hormone LH identifié dans l'échantillon est inférieur à un niveau de seuil d'hormone LH, et le dispositif de test (1) définit le niveau de seuil de gonadotrophine hCG pour identifier la grossesse à un second niveau de seuil de gonadotrophine hCG si le niveau d'hormone LH identifié dans l'échantillon est supérieur à un niveau de seuil d'hormone LH, dans lequel le premier niveau de seuil de gonadotrophine hCG est inférieur au second niveau de seuil de gonadotrophine hCG.

5. Dispositif de test (1) selon la revendication 4, dans lequel la différence entre les premier et second niveaux de seuil de gonadotrophine hCG est d'au moins 5 UI/L ou d'au moins 15 UI/L.

6. Dispositif de test (1) selon la revendication 3, dans lequel le niveau de seuil de gonadotrophine hCG pour identifier une grossesse est défini sur l'un parmi trois différents niveaux de seuil de gonadotrophine hCG ou plus en fonction du niveau d'hormone LH identifié dans l'échantillon.

7. Dispositif de test (1) selon l'une quelconque des revendications précédentes, dans lequel le dispositif de test (1) est destiné à identifier une grossesse dans le corps et à identifier une phase d'ovulation dans un cycle menstruel dans le corps,
dans lequel, si une grossesse n'est pas identifiée dans le corps, le dispositif de test (1) est configuré de manière à identifier une phase d'ovulation dans un cycle menstruel dans le corps si le niveau d'hormone LH identifié dans l'échantillon est supérieur à un niveau de seuil d'hormone LH.

8. Dispositif de test (1) selon l'une quelconque des revendications précédentes comprenant un écran d'affichage (36), dans lequel le dispositif de test est configuré de manière à présenter des informations sur l'identification de la grossesse à un utilisateur ou à une utilisatrice par l'intermédiaire de l'écran d'affichage (36).

9. Dispositif de test (1) selon l'une quelconque des revendications précédentes, dans lequel le dispositif de test (1) est un dispositif de test portatif.

10. Dispositif de test selon l'une quelconque des revendications précédentes comprenant une ou plusieurs bandes de test à écoulement latéral (10), dans lequel ladite une ou lesdites plusieurs parties de test sont comprises dans ladite une ou lesdites plusieurs bandes de test à écoulement latéral (10) .

11. Procédé d'identification d'au moins une grossesse dans un corps humain ou animal, le procédé comprenant les étapes ci-dessous consistant à :
identifier une première substance à analyser dans un échantillon biologique du corps, la première substance à analyser étant la gonadotrophine hCG ;
identifier une seconde substance à analyser dans l'échantillon biologique, la seconde substance à analyser étant l'hormone LH ; et
identifier une grossesse dans le corps si le niveau identifié de gonadotrophine hCG dans l'échantillon est supérieur à un niveau de seuil de gonadotrophine hCG, le niveau de seuil de gonadotrophine hCG variant en fonction du niveau d'hormone LH identifié dans l'échantillon.

12. Procédé selon la revendication 11, dans lequel le niveau de seuil de gonadotrophine hCG est modifié discrètement en fonction du niveau de la seconde substance à analyser identifié dans l'échantillon, et le procédé comprend les étapes ci-dessous consistant à :
identifier une grossesse dans le corps si le niveau identifié de gonadotrophine hCG dans l'échantillon est supérieur à un premier niveau de seuil de gonadotrophine hCG et si le niveau d'hormone LH identifié dans l'échantillon est inférieur au niveau de seuil d'hormone LH ; et
identifier une grossesse dans le corps si le niveau identifié de gonadotrophine hCG dans l'échantillon est supérieur à un second niveau de seuil de gonadotrophine hCG et si le niveau identifié de la seconde substance à analyser dans l'échantillon est supérieur à un niveau de seuil d'hormone LH,
dans lequel le premier niveau de seuil de gonadotrophine hCG est inférieur au second niveau de seuil de gonadotrophine hCG.

13. Procédé selon la revendication 12, dans lequel la différence entre les premier et second niveaux de seuil de gonadotrophine hCG est d'au moins 5 UI/L ou d'au moins 15 UI/L.

14. Procédé selon la revendication 11, dans lequel le niveau de seuil de gonadotrophine hCG est modifié discrètement en fonction du niveau d'hormone LH identifié dans l'échantillon, et le niveau de seuil de gonadotrophine hCG pour identifier une grossesse est défini sur l'un parmi trois différents niveaux de seuil de gonadotrophine hCG ou plus en fonction du niveau d'hormone LH identifié dans l'échantillon.

15. Procédé selon l'une quelconque des revendications 11 à 14, dans lequel le procédé est destiné à identifier une grossesse dans le corps et à identifier une phase d'ovulation dans un cycle menstruel dans le corps, le procédé comprenant en outre l'étape ci-dessous consistant à :
si une grossesse n'est pas identifiée dans le corps, identifier une phase d'ovulation dans un cycle menstruel dans le corps si le niveau d'hormone LH identifié est supérieur à un niveau de seuil d'hormone LH.
